# EUROPEAN PATENT SPECIFICATION

(11) **EP 2 709 969 B1**
(45) Date of publication and mention of the grant of the patent: **10.04.2019**
(21) Application number: 12705732.1
(22) Date of filing: 26.01.2012
(51) Int. Cl.: C07B 41/00

(54) **OZONOLYSIS PROCESS AND SAFETY SYSTEM FOR AN OZONE ABSORBER COLUMN**
OZONOLYSEVERFAHREN UND SICHERHEITSSYSTEM FÜR EINE OZONABSORPTIONSSÄULE
PROCÉDÉ D'OZONOLYSE ET SYSTÈME DE SÉCURITÉ POUR COLONNE D'ABSORPTION À OZONE

(30) Priority: 28.01.2011 US 201161437583 P
(43) Date of publication of application: 26.03.2014
(73) Proprietor: Emery Oleochemicals LLC, Cincinnati, OH 45232 (US)
(72) Inventor: WALKER, Thomas, Chad, Independence, KY 41051 (US)
(74) Representative: HGF Limited
(86) International application number: PCT/US2012/022699
(87) International publication number: WO 2012/103317

(56) References cited:
- WO-A2-2007/072097
- US-A1- 2009 118 498
- AYMAN D. ALLIAN ET AL: "The Development of Continuous Process for Alkene Ozonolysis Based on Combined in Situ FTIR, Calorimetry, and Computational Chemistry", ORGANIC PROCESS RESEARCH & DEVELOPMENT, vol. 15, no. 1, 24 November 2010 (2010-11-24), pages 91-97, XP55035596, ISSN: 1083-6160, DOI: 10.1021/op100249z
- "Explosions dans une usine pharmaceutique les 13/08/2003 et 09/08/2004", , 1 June 2009 (2009-06-01), pages 1-6, XP55035634, Retrieved from the Internet: URL:http://www.aria.developpement-durable. gouv.fr/ressources/fd_25337_35822_linz_ih_ fr_vfinal.pdf [retrieved on 2012-08-16]
- "elements22", , 1 January 2008 (2008-01-01), pages 1-32, XP55035636, Retrieved from the Internet: URL:http://www.imm-mainz.de/fileadmin/uplo ad/dateien/elements22_en.pdf [retrieved on 2012-08-16]

## Description

### FIELD OF THE INVENTION

The present invention relates to a process for producing an ozonide from an ethylenically unsaturated compound.

### BACKGROUND OF THE INVENTION

Azelaic acid and pelargonic acid can be produced in commercial quantities via an oxidative cleavage of an alkenyl unit (i.e., double bond between two carbon atoms) in oleic acid. For example, azelaic acid has been prepared from oleic acid by oxidation with chromium sulfate. However, because stoichiometric use of chromium reagents is undesirable, a more efficient oxidation approach utilizing ozone was developed.

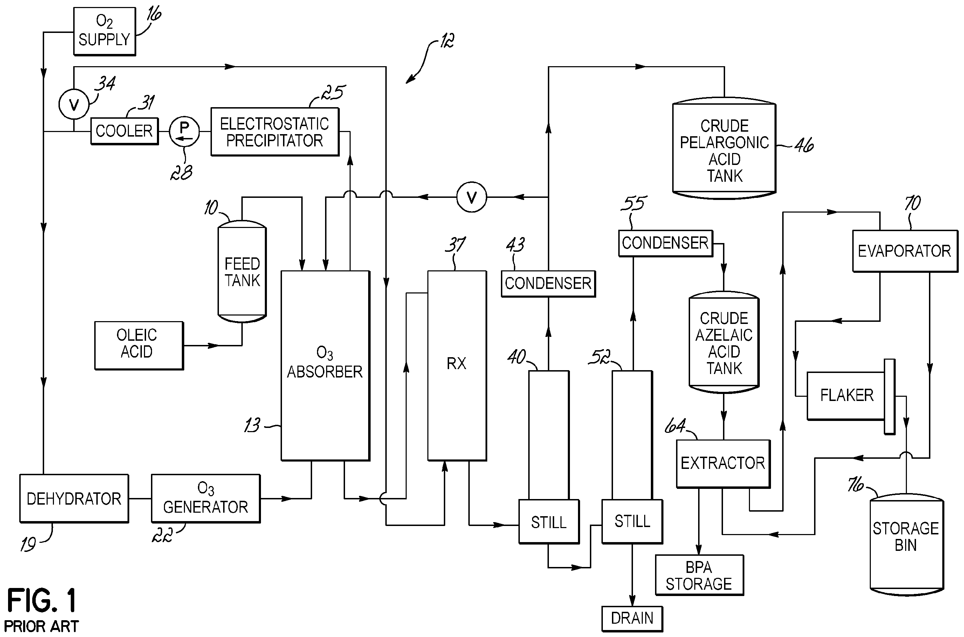

The basic process is best understood by referring to the description in the accompanying FIG. 1, which is a diagrammatic flow chart indicating the pieces of equipment used and their relationship in the ozonolysis process. This process involves reacting an ethylenically unsaturated compound, such as oleic acid, with ozone in an absorber 13 to form an ozonide. The ozone is provided by a continuous closed system 12 that recirculates and recycles the ozone enriched gas. The ozonide is transferred from the absorber 13 to one or more reaction chambers 37 where it is decomposed in the presence of additional oxygen and optional ozone to form a mixture of compounds including monobasic and dibasic acids, the mixture being referred to as mixed oxidation products (MOP). Monobasic acids and dibasic acids are then separated and individually processed in a series of stills 40 and 52, condensers 43 and 55, extractor 64, and evaporators 70 to remove compounds and undesired fractions among the range of molecular weights of monobasic and dibasic acids produced. The separated and purified monobasic and dibasic acids are then stored in storage tanks 46, 76.

Ozonized gas is fed into the absorber 13 by a continuous closed system 12 through which the gas circulates. In the closed system 12, the gas is recycled, i.e., the gas is reconditioned for reuse multiple times in the absorber 13. The closed system 12 reconditions the gas by removing organic compounds and water from the gas, restoring the desired oxygen concentration, and generating the desired concentration of ozone.
The closed system 12 maintains the desired predetermined oxygen concentration by bleeding off a small portion of the spent gas and replacing the bled off portion with fresh oxygen gas from an oxygen supply 16. The gas is then passed through a dehydrator 19 before being transferred to an ozone generator 22 which utilizes electricity to generate ozone. From the ozone generator 22, the ozone and oxygen mixture passes to the absorber 13 in which its ozone content is absorbed by the oleic acid as further explained below. From the absorber 13, the oxygen gas, now substantially devoid of ozone, passes to an electrostatic precipitator 25, which removes any contaminating fine mist organic compounds that may have been picked up in the absorber 13. The decontaminated oxygen gas then passes from the electrostatic precipitator 25 through a compression pump 28 to a cooler 31 and then returns back to the dehydrator 19, in which substantially all remaining moisture is removed to complete a pass through the closed system 12. Between the cooler 31 and dehydrator 19, a portion of the gas may be bled from the closed system 12 through a valve to one or more reaction chambers 37 for reaction with the ozonide to form the MOP.

While the process and apparatus described above generates organic acids such as azelaic and pelargonic acids, from longer chain unsaturated organic acids such as oleic acid, deficiencies exist with respect to personnel safety, system efficiencies and equipment longevity. In particular, the reaction of the ozone with ethylenically unsaturated compounds such as oleic acid to form ozonides is exothermic. Moreover, ozonides are very unstable and can spontaneously decompose and explode if the temperature is not carefully maintained below certain thresholds, such as by controlling the concentration of the reactants and the temperature of the absorber column with coolers. Temperatures within the absorber column can rise above safe operating values due to, for example, a failure in the cooling system or a change in the flow rate or concentration of one or both of the ozone containing gas and the unsaturated fatty acid. To decrease the risk of an accidental explosion of the ozonide, a system that considers the operating temperatures, the flow rates, pressures, and volumes for the reactants and products, and then responds to those parameters in the event they fall outside acceptable ranges is needed to improve the safety of the process.
In Org. Process Res. Dev. 2011, 15(1), pages 91 to 97 two continuous apparatuses are disclosed that are capable of safely scaling the highly energetic ozonolysis reaction to multikilogram scale where flow chemistry allowed excellent temperature control minimizing the inventory of the highly unstable ozonide intermediate.
WO 2007/072097 discloses a flow-type laboratory scale ozonolysis apparatus comprising a reservoir, a feed pump, a mixing element with two inlets and an outlet, a reactor unit and a pressure-adjusting means, all connected into a flow path. The ozonolysis apparatus further comprises an ozone source, as well as a dispensing valve transmitting a gas stream only in a single direction and installed between the ozone source and one of the inlets of the mixing element. The feed pump of the ozonolysis apparatus is a liquid pump generating a constant volume rate, the reservoir contains at least the substance, as a solute, to be subjected to the ozonolysis reaction and the reactor unit consists of first and second reactor zones differing in function from one another. In the flow path, the outlet of the first reactor zone is connected to the inlet of the second reactor zone. Furthermore, an inlet for feeding in substances is inserted into the flow path between the reactor zones, and the pressure-adjusting means is installed into the flow path after the reactor unit and is provided with an electrically governed control.
"elements22", Evonik Science Newsletter 2008 mentions an ozonolysis process of cyclohexene in which the diverse reaction parameters (temperature, flow rates and mixing ratios) were precisely controlled.
US 2009/118498 discloses a method for increasing ozone concentration in a liquid including: providing a gas having ozone; introducing the ozone-containing gas into a liquid, wherein the liquid and ozone combination has a temperature between 0.8 and 1.5 times the critical temperature of ozone; and increasing isothermally, the pressure of the ozone-containing gas above the liquid to 0.3 to 5 times the critical pressure of ozone so as to increase the ozone concentration in the liquid. The temperature is expressed in absolute units (Kelvin or Rankin). The method can be used for removing ozone from a gas or for purifying ozone. The liquid having a high ozone concentration can be used for ozonolysis of a substrate.

### SUMMARY

The invention refers to a process for producing an ozonide in accordance with claim 1. In particular, the invention refers to a process for producing an ozonide from an ethylenically unsaturated carboxylic acid having between 8 and 30 carbon atoms and one or more unsaturated carbon to carbon double bonds comprising the steps of
contacting an ozone enriched gas with the unsaturated carboxylic acid in an absorber column (110) having a trough (126) at the top (128) and a pot (130) at the bottom (132) having separate stages to form an ozonide; and
operating a safety system comprising:
monitoring an operating condition wherein the operating condition includes at least one of an operating temperatures within the column, the flow of ozone enriched gas having an ozone concentration between 0.5% to 12% through the column, the operating pressure of ozone enriched gas in the column, the flow of liquid reactant through the column, and the flow of product out of the column and assigning a value to the monitored operating condition; and wherein each operating condition is independently monitored;
comparing the operating condition value with a threshold value for the at least one condition;
affecting the formation of the product in response to the operating condition reaching the threshold value;
wherein the operating condition value is a temperature in the absorber column and the threshold value is a first threshold temperature between 37°C (100°F) and 60°C (140°F);
and wherein a sensor monitors the temperature within the column and a switch linked to the sensor stops the supply of ozone enriched gas to the absorber column by closing an ozone enriched gas valve and by causing the ozone generators to shut down by switching off supply of electricity to the generators if the temperature at any of the stages in the absorber column, or in the trough or in the pot is above the first threshold temperature value that is between 37°C (100°F) and 60°C (140°F);
and wherein the threshold value further includes a second threshold temperature that is greater than the first threshold temperature, and the second threshold temperature is between 43°C (110°F) and 66°C (150°F), and wherein a second independent temperature monitor detects the temperature of stages within the column and if the temperature exceeds or reaches the second threshold temperature but is less than the critical temperature a switch linked to the second temperature sensor causes the ozone generators to shut down, the ozone enriched gas valve supplying the absorber column to close, and water valves are opened to the stage where the second threshold temperature was exceeded and the formation of the product in that stage in response to the temperature reaching the second threshold value is affected by direct addition of water into the absorber column.

Also described herein is an improved process for producing a carboxylic acid mixture comprising a saturated mono carboxylic acid and a saturated dicarboxylic acid.

The process includes generating an ozone enriched gas in an ozone generator and introducing the ozone enriched gas to an absorber column. There, the ozone enriched gas is contacted with an unsaturated carboxylic acid feed in the absorber to form an ozonide. The ozonide is contacted with an oxygen enriched gas in a reactor to obtain the carboxylic acid mixture. The carboxylic acid mixture is optionally purified. The process further includes a safety system that monitors operating conditions of the absorber column and responds to those conditions which are able to disrupt the formation of ozonide or generate other materials capable of causing a safety risk should the operating conditions exceed a threshold value.

### BRIEF DESCRIPTION OF THE DRAWINGS

FIG. 1 is a schematic representation of a prior art oleic acid ozonolysis system.
FIG. 2 is a schematic representation of the safety system in an exemplary absorber column.

### DETAILED DESCRIPTION OF THE INVENTION

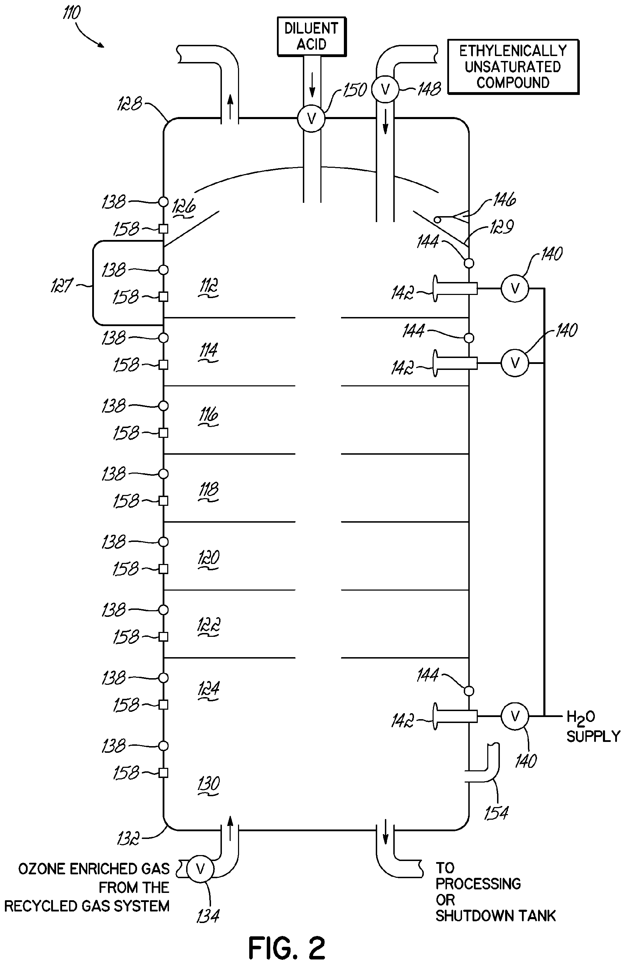

The gas recycling system is used to circulate and recondition depleted gas from an absorber column in which an ozonizing reaction is conducted. The recycle gas system is useful to recondition the depleted gas from the absorber and as shown in Fig. 2, provides a significant improvement in efficiency and safety over the recycle gas system shown in Fig. 1.

This invention is directed to a safety system that includes sensors, switches, valves, and rupture disks located in the vicinity of an ozone absorber column used in the process of adding ozone across a double bond of an unsaturated organic compound, such as oleic acid. The ozonation reaction forms an ozonide of the unsaturated organic compound at the unsaturated bond. The ozonide leaves the absorber column and is oxidized in a series of oxidation reactions to yield a mixture of a dibasic acid, such as azaleic acid, and a monobasic acid, such as pelargonic acid. The desired monobasic and dibasic acids may be purified from by products and each other in a series of distillation and crystallization steps. The reaction product, i.e., the ozonide, is an extremely unstable species, and can create an explosion risk if the ozonide temperature in the absorber column exceeds prescribed limits. To minimize the hazard to personnel and equipment in the vicinity of the absorber column and to decrease the down-time associated with having to completely shut down and restart the process, the safety system includes a plurality of valve and switch assemblies arrayed at various locations contiguous to the column to interrupt reactant and product flows, as well as to introduce a water quench into the column under certain conditions. In addition, the safety system includes mechanisms to safely relieve excessive pressure in the absorber column that could result in an explosion.

The conditions that are monitored in the absorber column 110 include the operating temperatures within the absorber column 110, the flow of reactants through the column 110 (e.g., ethylenically unsaturated compound, such as oleic acid, ozone carried by an oxygen gas (ozone enriched gas), or both), the flow of the product (e.g., ozonide) out of the column, and the operating pressure in the absorber column 110. Each condition is typically monitored independently of the other conditions. If any one of these conditions is observed to meet or exceed the threshold values described in further detail below, then an aspect of the safety system is activated, shutting down the column and decreasing the risk of an explosion.

The absorber column 110 has separate stages 112, 114, 116, 118, 120, 122, and 124 that optimize the reaction conditions for the reactants, e.g. an unsaturated fatty acid such as oleic acid and ozone, to produce an ozonide. The exemplary absorber column 110 described herein has seven such stages and further includes a trough 126 at the top 128 of the absorber column 110 and a pot 130 at the bottom 132. The unsaturated fatty acid enters the trough 126 at the top 128 of the absorber column 110 and flows down the column 110 through the separate stages 112, 114, 116, 118, 120, 122, 124. The ozone enriched gas has an ozone concentration in the range between 0.5% to 12% ozone, enters near the bottom 132 of the absorber column 110 and travels up the absorber column 110 through the separate stages 112, 114, 116, 118, 120, 122, 124, counter-current to the flow of the unsaturated fatty acid. While the exemplary absorber column 110 has seven stages 112, 114, 116, 118, 120, 122, 124 it is understood that absorber columns with greater than or less than seven stages are within the scope of the invention. Accordingly, the number of components for the safety system such as the number of sensors, switches, valves, and rupture disks may similarly be varied and are within the scope of the invention.

Due to the instability of the mixture of ozone enriched gas and the ethylenically unsaturated compounds, as well as their reaction products (i.e., the ozonide and its intermediates and by products), the temperature in the reaction column no must not be allowed to exceed a critical temperature above which the risk of explosion becomes too great. With this safety concern in mind, the first element of the safety system described herein includes a sensor that monitors the temperature within the column and a switch linked to the sensor stops the supply of the ozone enriched gas to the absorber column no in the event that the sensor detects a temperature at any of the stages 112, 114, 116, 118, 120, 122, 124 of the absorber column 110 that is above a first threshold temperature but is less than the critical temperature. The switch causes the ozone generators to shut down by switching off supply of electricity to the generators thereby stopping the production of ozone in the generators. The switch also causes the ozone enriched gas valve 134, which supplies the absorber column 110 with ozone enriched gas, to close. In one embodiment, the first threshold temperature value is between 37°C (100°F) and 60°C (140°F) as measured at any of the stages 112, 114, 116, 118, 120, 122, 124 in the absorber column 110, in the trough 126, or in the pot 130. Alternatively, the first threshold temperature value is between 43°C (110 °F) and 54°C (130°F). As another alternative, the threshold temperature value is between 49°C (120°F) and 52°C (125°F).

In the exemplary absorber column 110, the first threshold temperature is monitored and the response is controlled by first temperature monitor 138 located at each stage 112, 114, 116, 118, 120, 122, 124 of the column 110. The first temperature monitor 138 includes a sensor to measure the temperature and a switch to effect the response as needed. The sensor and switch may be included in a single device or may be in discrete devices that are in communication with each other. The first temperature monitors 138 are set to detect if the temperature in any one of the stages 112, 114, 116, 118, 122, 124 of the column 110, or in the trough 126 or pot 130 reaches or exceeds the first threshold temperature. If the first threshold temperature is reached or exceeded as measured by the first temperature monitor 138, this monitor 138 will send a signal to automatically shut down the ozone generators (not shown) by switching off the supply of electricity to the ozone generator and also to close the ozone enriched gas valve 134, thereby preventing a runaway exothermic reaction that could result in an explosion.
The first temperature monitor 138 closes an electrical contact in the switch portion of the monitor 138 when the sensor portion detects a temperature in the column 110 that exceeds the first threshold temperature. The ozone enriched gas valve 134 closes automatically in response to the signal from the first temperature monitor 138. The ozone enriched gas valve 134 may be opened and closed by a control valve, such as an air pressure control valve as is known to those of ordinary skill in the art, that is linked to the first temperature monitor 138.

The safety system also includes a second temperature monitor 144 that is independent of the first temperature monitor 138. The second temperature monitor 144 detects the temperature of certain stages of the absorber column, such as stages 112, 122, or 124 in the exemplary column, and if the temperature exceeds a second threshold temperature that is greater than the first threshold temperature but less than the critical temperature, all ozone generators will shut down, the ozone enriched gas valve 134 supplying the absorber column 110 will close as described above, and water valves 140 will open to that corresponding stage wherein the second threshold temperature was exceeded, injecting water through a nozzle 142 to cool the absorber column 110 and to quench the reaction in that stage. The second threshold temperature value is between 43°C (110°F) and 66°C (150°F). Alternatively, the second threshold temperature value is between 49°C (120°F) and 60°C (140°F). As another alternative, the second threshold temperature value is between 52°C (125°F) and 57°C (135°F).

When the temperature in the monitored stages reaches the second threshold temperature, the risk of a runaway reaction in that stage which can result in explosion is substantially increased. Typically, when the risk of a runaway reaction in a reactor such as an absorber column is too high, i.e., reaches the second threshold, the response is to shut off the supply of reactants to the reactor and to cool the reactor such as with the external application of a coolant. For most runaway or near runaway chemical reactions, cooling agents are not directly added to the reactant because of the risk that the cooling agent could either react with the reactants or rapidly convert to a gas (or steam, as is the case with water) thereby rapidly increasing the pressure in the vessel and increasing the risk of an explosion. However, in the reaction occurring in the absorber column, it was surprisingly observed that the direct addition of water quenches the reaction and decreases the likelihood of a runaway reaction. Accordingly, water is injected into the absorber column with the reactants when the temperature in a stage of the absorber column reaches or exceeds a second threshold.

Water is injected at a rate to quench the reaction and continues to be injected until the temperature reaches the first threshold temperature or below, at which point water injection is stopped by closing the water valves 140. Typically, the operator of the absorber column 110 will stop the injection of the water by closing the water valves 140. However, automatic systems, such as a computer controlled system (not shown), can also be used. The material in the column 110, i.e., water, reactants, and products, may then be sent to a catch tank (not shown) and processed for recycling back through the system to complete the reaction.

In addition to the first and second temperature monitors 138, 144 described above, the safety system also monitors and responds to changes in the flow of reactants in the absorber column. One of the reactants is the ozone enriched gas that flows from the recycled gas system to the absorber column. The ozone enriched gas flow safety component is activated if either or both of the pressure or volume of the ozone enriched gas drops below their respective threshold values. An exemplary ozone enriched gas pressure threshold is in the range between 0 kPa (0 psig) to 103kPa (15 psig), or, alternatively, in the range between 34 kPa (5 psig) and 68 kPa (10 psig). If the ozone enriched gas pressure drops to the ozone enriched gas pressure threshold or below, then the ozone generators will shut down and close the ozone enriched gas valve 134 feeding the absorber column 110. The ozone enriched gas pressure is measured by pressure sensors located upstream from the ozone generators. Any gas pressure sensor as known to those skilled in the art may be used. A similar result will follow if the ozone enriched gas flow falls below the ozone enriched gas volume threshold. The ozone enriched gas flow is measured at the inlet of the ozone generator with a flow meter that has a low level trip, i.e., is tripped when the flow drops below the threshold value. Any gas flow meter as known to those skilled in the art may be used.

In addition to the safety features described above, the safety system described herein also monitors and responds to the flow of liquid reactant through the column. As the liquid reactant and ozone enriched gas pass through the column, some liquid reactant will become entrained as liquid droplets in the ozone enriched gas. The amount of liquid reactant entrained in the ozone enriched gas will increase if the liquid reactant begins backing up in any of the lower stages of the column. Therefore, increased levels of entrained liquid reactant may be used as an indicator of the flow of liquid reactants through the column.

The trough 126 is part of a system at the top of the column that removes the entrained liquid reactant from the ozone enriched gas. The trough 126 collects the liquid reactant that has been removed from the ozone enriched gas. The collected liquid reactant is then drained to a lower stage of the column. The drain 127 has a limited capacity to remove collected liquid reactant in the trough 126. Generally, the drain 127 may remove the volume of liquid reactant collected from the ozone enriched gas under normal operating conditions without the liquid reactant pooling in the trough.
However, if the volume of liquid reactant entrained in the ozone enriched gas increases, such as will occur if the liquid reactant becomes backed up on a lower stage of the column, then the drain 127 will be unable to remove the increased collected volume of liquid reactant, which will begin to pool in the trough 126. In one embodiment, the safety system monitors the volume of liquid reactant in the trough 126.

In one embodiment, the volume of liquid reactant in the trough 126 is monitored. If the depth of liquid reactant in the trough 126 meets or exceeds a threshold value, referred to herein as the liquid reactant threshold, a sensor 146 will detect the increased depth of the liquid reactant and generate a signal to shut down the ozone generators as described above, close the ozone enriched gas valve 134, close the ethylenically unsaturated compound valve 148 supplying the column 110, and also close the diluent feed valve 150 used to control the viscosity of the mixture of the ozone enriched gas with the liquid reactant. The liquid reactant threshold can vary depending on the characteristics of the column 110, such as the volume of the trough, size of the drain, etc. In another embodiment, the liquid reactant threshold is a depth of liquid reactant in the trough 126 that is greater than the normal operating depth of liquid reactant in the trough. The liquid reactant threshold can be an increase of at least 5% of the normal operating depth of the liquid reactant in the trough, depending on the characteristics of the column. The change in depth correlates to a change in the volume of liquid reactant in the trough of at least 5% over the normal operating volume of the liquid reactant in the trough, depending on the characteristics of the column.

In an exemplary embodiment, the sensor 146 begins indicating an increasing depth of liquid reactant as soon as the liquid reactant touches the sensor 146, which can be located 0.6 cm (0.25 inches) to 12.7 cm (5 inches) above the bottom 129 of the trough 126. In this embodiment, the threshold is triggered when the sensor detects a change in the depth of the liquid reactant in the range from 0.6 cm (0.25 inches) to 3.8 cm (1.5 inches) above the normal operating depth of the liquid reactant, or a 5% to a 600% change in the volume of liquid reactant in the trough collected during normal operating conditions.

Any sensor 146 that is able to detect changes in the volume of the liquid reactant may be used, such as a float mechanism, as are known to those of ordinary skill in the art. The term "liquid reactant" is understood to include the mixture of ethylenically unsaturated compound, such as oleic acid, and its reaction products and intermediaries as well as a diluent. Such diluent includes, but is not limited to, saturated short chain acids such as acetic acid, butanoic acid, caproic acid, heptanoic acid, caprylic acid, pelargonic acid, and capric acid; esters such as ethyl acetate and butyl acetate; and alkanes such as hexane, octane, and decane. However, the use of pelargonic acid is recommended because, as an end product of the process, it does not interfere with the operation of the circulating oxygen system and requires no separate distillation. In other words, since pelargonic acid is one of the end products of the process, it is an ideal diluent.

Similarly, the safety system also monitors and responds to the flow of the product out of the pot. The term "product" is understood to include the ozonide, unreacted liquid reactants, and diluent. This aspect is monitored as part of the safety system to prevent overfilling the pot 130 and possibly backing up in the column 110 of the product, which can result in an explosion. The flow of product out of the pot 130 may be determined by monitoring the liquid level of the product in the pot 130. The liquid level of the product in the pot may be measured with a differential pressure sensor 154 that measures the head of the liquid, or with a float switch similar to the float switch 146 described above. If the level of product meets or exceeds a threshold level in the pot 130, the safety system will shut down the ozone generators, close the ozone enriched gas valve 134 supplying the absorber column 110 with ozone enriched gas, close the unsaturated carboxylic acid valve 148 supplying the column 110, and also close the diluent feed valve 150. In an exemplary embodiment, sensor 154 in Fig. 2 is used detect if level of product in the pot reaches a product threshold level of 1/3 of the height of the pot 130 or a height of 56 cm (22 inches) from the bottom of the pot having a diameter at its widest point of 86 cm (34 inches). It is understood that the product threshold level can vary depending on the characteristics of the column 110.

Another component of the safety system described herein are mechanisms for relieving excessive pressure that could result in an explosion in the absorber column. To this end, the safety system also includes rupture disks 158 fitted throughout the absorber column no that are designed to burst if the pressure anywhere in the column no exceeds a first threshold operating pressure that is at or below maximum safe operating pressure for the absorber column no. In one embodiment, the first threshold pressure is in the range between 138 kPa (20 psig) to 414 kPa (60 psig), or more preferably in the range between 241 kPa (35 psig) to 310 kPa (45 psig). The rupture disks can be formed as known in the art from a disk of material, such as a metal, and designed to burst at specified pressure. When the rupture disks burst, the vented gases and product are discharged into a catch tank. In addition, piping that is associated with the absorber column may also be fitted with rupture disks.

The mechanism for relieving excessive pressure may also include at least one relief valve (not shown) on the dryer (not shown) upstream of the ozone generators that is set to open if the pressure within the dryer exceeds a second threshold operating pressure. This prevents the pressure within the ozone enriched gas supply from being too great upon entry into the column 110. If the ozone enriched gas pressure is greater than the second threshold operating pressure, then the mixture of the reactants, i.e., ozone and oleic acid, could result in a runaway reaction and explosion. In the exemplary system described herein, the dryer pressure relief valve is set to open if the pressure at the dryer exceeds the second threshold pressure in the range between 68 kPa (10 psig) and 172 kPa (25 psig), or more preferably between 103 kPa (15 psig) 138 kPa (20 psig).

The safety system described herein is useful with an absorber column that generates ozonides of unsaturated acids in an ozonolysis system. As mentioned above, the safety system is particularly suited for use with an ozonolysis system that breaks down oleic acid into pelargonic acid and azelaic acid. However, the safety system may be useful with ozonolysis systems used to break down other unsaturated acids into component carbon chains which form monobasic and dibasic acids via the ozonolysis reaction. The unsaturated acids have between 8 and 30 carbon atoms and one or more unsaturated carbon to carbon bonds. The monobasic and dibasic acid products that result from the ozonolysis reaction are determined by the location of the one or more unsaturated carbon to carbon bonds in the unsaturated acid. The unsaturated acids may be isolated from biological sources, such as plants, animals, or microorganisms. Alternatively, the unsaturated acids may be isolated from petroleum sources and synthetic sources. Exemplary mono unsaturated acids and their respective potential oxidation products are included in the Table below.

| Carbons | Exemplary Unsaturated Fatty Acid | Exemplary Monobasic Product | Exemplary Dibasic Product |
|---|---|---|---|
| 10 | Obtusilic acid | Caproic acid | Succinic acid |
| 10 | Caproleic acid | Formic acid | Azelaic acid |
| 11 | Undecenoic acid | Formic acid | Sebacic acid |
| 12 | Lauric acid | Propionic acid | Azelaic acid |
| 14 | Myristoleic acid | Valeric acid | Azelaic acid |
| 16 | Palmitoleic acid | Heptanoic acid | Azelaic acid |
| 18 | Petroselinic acid | Lauric acid | Adipic acid |
| 18 | Oleic acid | Pelargonic acid | Azelaic acid |
| 18 | Vaccenic acid | Heptanoic acid | Hendecanedioic acid |
| 18 | Octadecenoic acid | Caproic acid | Dodecanedioic acid |
| 20 | Gadoleic acid | Undecanoic acid | Azelaic acid |
| 22 | Cetoleic acid | Undecanoic acid | Hendecanedioic acid |
| 22 | Erucic acid | Pelargonic acid | Brassylic acid |
| 24 | Selacholeic acid | Pelargonic acid | Pentadecanedioic acid |
| 26 | Hexacosenoic acid | Pelargonic acid | Heptadecanedioic acid |
| 30 | Tricosenoic acid | Pelargonic acid | Heneicosanedioic acid |

While the list above includes monounsaturated acids, it is understood that polyunsaturated acids could be utilized as well. The resulting monobasic acids and dibasic acids, and their respective derivatives, may be used for a number of different purposes such as in the preparation of lubricant base stocks, plasticizers, lacquers, herbicides, skin treatments, textile coning oils, flotation agents for mineral refining, fragrances, catalyst scavengers, corrosion inhibitors, metal cleaners, polymerization initiators, lithium complex greases, epoxy flexibilizers, thermosetting unsaturated polyester resins, polyamide hot melts, urethane elastomers, and elastomeric fibers, wire coatings and molding resins.

## Claims

1. A process for producing an ozonide from an ethylenically unsaturated carboxylic acid having between 8 and 30 carbon atoms and one or more unsaturated carbon to carbon double bonds comprising the steps of
contacting an ozone enriched gas with the unsaturated carboxylic acid in an absorber column (110) having a trough (126) at the top (128) and a pot (130) at the bottom (132) having separate stages to form an ozonide; and
operating a safety system comprising:
monitoring an operating condition wherein the operating condition includes at least one of an operating temperatures within the column, the flow of ozone enriched gas having an ozone concentration between 0.5% to 12% through the column, the operating pressure of ozone enriched gas in the column, the flow of liquid reactant through the column, and the flow of product out of the column and assigning a value to the monitored operating condition; and wherein each operating condition is independently monitored;
comparing the operating condition value with a threshold value for the at least one condition;
affecting the formation of the product in response to the operating condition reaching the threshold value;
wherein the operating condition value is a temperature in the absorber column and the threshold value is a first threshold temperature between 37°C (100°F) and 60°C (140°F);
and wherein a sensor monitors the temperature within the column and a switch linked to the sensor stops the supply of ozone enriched gas to the absorber column by closing an ozone enriched gas valve and by causing the ozone generators to shut down by switching off supply of electricity to the generators if the temperature at any of the stages in the absorber column, or in the trough or in the pot is above the first threshold temperature value that is between 37°C (100°F) and 60°C (140°F); and
wherein the threshold value further includes a second threshold temperature that is greater than the first threshold temperature, and the second threshold temperature is between 43°C (110°F) and 66°C (150°F), and wherein a second independent temperature monitor detects the temperature of stages within the column and if the temperature exceeds or reaches the second threshold temperature but is less than the critical temperature a switch linked to the second temperature sensor causes the ozone generators to shut down, the ozone enriched gas valve supplying the absorber column to close, and water valves are opened to the stage where the second threshold temperature was exceeded and the formation of the product in that stage in response to the temperature reaching the second threshold value is affected by direct addition of water into the absorber column.

2. The process of claim 1 wherein the safety system also monitors the flow of ozone enriched gas to the absorber column from the recycled gas system and the flow safety components is activated if either or both of the pressure or volume of the ozone enriched gas drops below threshold values, wherein the operating condition value is a threshold pressure measured by a gas pressure sensor upstream from the ozone generators and/or a threshold volume of the ozone enriched gas measured by a gas flow meter at the inlet of the ozone generator, wherein the threshold gas pressure value is an ozone enriched gas pressure between 0 kPa (0 psig) and 103 kPa (15 psig); and wherein if the gas pressure drops to the threshold value or below the ozone generators will shut down and close an ozone enriched gas valve to stop the ozone enriched gas supply to the absorber column.

3. The process of claim 1 or 2 wherein the safety system also monitors the flow of liquid reactant through the absorber column by monitoring the volume of liquid reactant in the trough, and wherein the operating condition value is a threshold value of the depth of liquid reactant in the trough.

4. The process of claim 3 wherein the flow of liquid reactant is monitored by a sensor to detect the volume of the liquid reactant in the trough of the column; and wherein the threshold value is an increase of the depth of liquid reactant in the trough by at least 5% of the normal operating depth of liquid in the trough of the absorber column.

5. The process of one of claims 3 or 4 wherein if the threshold value is met or exceeded a signal is generated by the sensor to shut down the ozone generators, close the ozone enriched gas valve, close the ethylenically unsaturated compound valve, and close the diluent feed valve.

6. The process of one of claims 1 to 5 wherein the safety system also monitors the flow of product, wherein the product is ozonide, undiluted liquid reactants and diluent and wherein the operating condition value is monitored by measuring the level of liquid in the pot of the column, wherein the threshold value is a product level of 1/3 of the height of the pot, and wherein if the level of product meets or exceeds the threshold the formation of ozonide is affected by the safety system shutting down the ozone generators, closing the ozone enriched gas valve, closing the unsaturated carboxylic acid valve, and closing the diluent feed valve.

7. The process of one of claims 1 to 6 wherein the safety system includes mechanisms for relieving excessive pressure in the absorber column if the pressure in the column exceeds a first threshold operating pressure, wherein rupture disks designed to burst if the pressure in the column exceeds the first threshold operating pressure are fitted throughout the absorber column, and wherein the first threshold operating pressure is at or below the safe operating pressure for the absorber column.

8. The process of claim 7 wherein the first threshold operating pressure is between 137900 pascal (20 psig) and 413700 pascal (60 psig).

9. The process of one of claims 1 to 8 wherein the safety system includes a mechanism for relieving excessive pressure upstream of the ozone generators and including at least one pressure relief valve on the dryer, wherein the mechanism is set to open if the pressure of the ozone enriched gas prior to the introduction into the absorber column exceeds a second threshold operating pressure.

10. The process of claim 9 wherein the pressure of the ozone enriched gas is relieved by the opening of a pressure relief valve and wherein the threshold for relieving the pressure is between 68950 pascal (10 psig) and 172400 pascal (25 psig).

11. The process of any one of claims 1 to 10 wherein the absorber has 7 stages, wherein a first temperature monitor is located at each stage, and wherein a second temperature monitor is located in 3 stages (112, 122, 124).

12. The process of any one of claims 1 to 11 wherein the unsaturated carboxylic acid is obtusilic, caproleic, undecenoic, lauric, myristoleic, palmitoleic, petroselinic, oleic, vaccenic, octadecanoic, gadoleic, cetoleic, erucic, selacholeic, hexacosenoic or tricosenoic acid,

13. The process of one of claims 1 to 12 wherein the unsaturated carboxylic acid is oleic acid.

## Patentansprüche

1. Verfahren zum Produzieren eines Ozonids aus einer ethylenisch ungesättigten Carbonsäure mit zwischen 8 und 30 Kohlenstoffatomen und einer oder mehreren ungesättigten Kohlenstoff-zu-Kohlenstoff-Doppelbindungen, der die folgenden Schritte beinhaltet:
Inkontaktbringen eines ozonangereicherten Gases mit der ungesättigten Carbonsäure in einer Absorberkolonne (110) mit einem Trog (126) an der Oberseite (128) und einem Topf (130) an der Unterseite (132) mit getrennten Stufen, um ein Ozonid zu bilden; und
Betreiben eines Sicherheitssystems, das Folgendes beinhaltet:
Überwachen einer Betriebsbedingung, wobei die Betriebsbedingungen mindestens eines von einer Betriebstemperatur innerhalb der Kolonne, dem Fluss von ozonangereichertem Gases mit einer Ozonkonzentration zwischen 0,5 % und 12 % durch die Kolonne, dem Betriebsdruck des ozonangereicherten Gases in der Kolonne, dem Fluss von flüssigem Reaktanten durch die Kolonne und dem Fluss von Produkt aus der Kolonne heraus und Zuordnen eines Wertes auf die überwachte Betriebsbedingung; und wobei jede Betriebsbedingungen unabhängig überwacht wird;
Vergleichen des Betriebsbedingungswertes mit einem Schwellenwert für die mindestens eine Bedingung;
Beeinflussen der Bildung des Produkts als Reaktion auf das Erreichen des Schwellenwerts der Betriebsbedingung;
wobei der Betriebsbedingungswert eine Temperatur in der Absorberkolonne ist und der Schwellenwert eine erste Schwellentemperatur zwischen 37 °C (100 °F) und 60 °C (140 °F) ist; und wobei ein Sensor die Temperatur innerhalb der Kolonne überwacht und ein mit dem Sensor gekoppelter Schalter die Speisung von ozonangereichertem Gas an die Absorberkolonne durch Schließen eines Ventils für das ozonangereicherte Gas und Bewirken des Ausschaltens der Ozongeneratoren durch Abschalten der Speisung von Elektrizität an die Generatoren, falls die Temperatur in einer der Stufen in der Absorberkolonne oder in dem Trog oder in dem Topf über dem ersten Schwellentemperaturwert, d. h. zwischen 37 °C (100 °F) und 60 °C (140 °F), liegt, unterbricht; und
wobei der Schwellenwert ferner eine zweite Schwellentemperatur umfasst, die größer als die erste Schwellentemperatur ist, und die zweite Schwellentemperatur zwischen 43 °C (110 °F) und 66 °C (150 °F) liegt, und wobei eine zweite unabhängige Temperaturüberwachungseinheit die Temperatur der Stufen innerhalb der Kolonne detektiert und, falls die Temperatur die zweite Schwellentemperatur überschreitet oder erreicht, aber geringer als die kritische Temperatur ist, ein Schalter, der mit dem zweiten Temperatursensor gekoppelt ist, das Ausschalten der Ozongeneratoren, das Schließen des Ventils für das ozonangereicherte Gas, das die Absorberkolonne speist,
und das Öffnen von Wasserventilen an die Stufe, in der die zweite Schwellentemperatur überschritten wurde und die Bildung des Produkts in der Stufe als Reaktion auf das Erreichen der Temperatur des zweiten Schwellenwerts durch direkte Hinzufügung von Wasser in die Absorberkolonne beeinflusst wird, bewirkt.

2. Verfahren gemäß Anspruch 1, wobei das Sicherheitssystem auch den Fluss des ozonangereicherten Gases und die Absorberkolonne von dem Recyclegassystem überwacht und die Flusssicherheitskomponenten aktiviert werden, falls eines oder beides von dem Druck oder Volumen des ozonangereicherten Gases unter die Schwellenwerte abfällt, wobei der Betriebsbedingungswert ein Schwellendruck, der von einem Gasdrucksensor stromaufwärts von den Ozongeneratoren gemessen wird, und/oder ein Schwellenvolumen des ozonangereicherten Gases, das von einem Gasflussmesser an dem Einlass des Ozongenerators gemessen wird, ist, wobei der Schwellengasdruckwert ein Druck des ozonangereicherten Gases zwischen 0 kPa (0 psig) und 103 kPa (15 psig) ist; und wobei, falls der Gasdruck auf den Schwellenwert oder unter diesen abfällt, die Ozongeneratoren ausschalten und ein Ventil für das ozonangereicherte Gas schließen werden, um die Speisung des ozonangereicherten Gases an die Absorberkolonne zu unterbrechen.

3. Verfahren gemäß Anspruch 1 oder 2, wobei das Sicherheitssystem auch den Fluss von flüssigem Reaktanten durch die Absorberkolonne durch Überwachen des Volumens von flüssigem Reaktanten in dem Trog überwacht und wobei der Betriebsbedingungswert ein Schwellenwert der Tiefe von flüssigem Reaktanten in dem Trog ist.

4. Verfahren gemäß Anspruch 3, wobei der Fluss von flüssigem Reaktanten durch einen Sensor überwacht wird, um das Volumen des flüssigen Reaktanten in dem Trog der Kolonne zu detektieren; und wobei der Schwellenwert eine Vergrößerung der Tiefe von flüssigem Reaktanten in dem Trog um mindestens 5 % der normalen Betriebstiefe von Flüssigkeit in dem Trog der Absorberkolonne ist.

5. Verfahren gemäß einem der Ansprüche 3 oder 4, wobei, falls der Schwellenwert erfüllt oder überschritten wird, von dem Sensor ein Signal generiert wird, um die Ozongeneratoren auszuschalten, das Ventil für das ozonangereicherte Gas zu schließen, das Ventil für die ethylenisch ungesättigte Verbindung zu schließen und das Ventil für die Verdünnungsmittelzufuhr zu schließen.

6. Verfahren gemäß einem der Ansprüche 1 bis 5, wobei das Sicherheitssystem auch den Fluss von Produkt überwacht, wobei das Produkt Ozonid, unverdünnte flüssige Reaktanten und Verdünnungsmittel ist und wobei der Betriebsbedingungswert durch Messen des Pegels von Flüssigkeit in dem Topf der Kolonne überwacht wird, wobei der Schwellenwert ein Produktpegel von 1/3 der Höhe des Topf ist und wobei, falls der Pegel von Produkt die Schwelle erfüllt oder überschreitet, die Bildung von Ozonid durch das Ausschalten der Ozongeneratoren durch das Sicherheitssystem, Schließen des Ventils für das ozonangereicherte Gas, Schließen des Ventils für die ungesättigte Carbonsäure und Schließen des Ventils für die Verdünnungsmittelzufuhr beeinflusst wird.

7. Verfahren gemäß einem der Ansprüche 1 bis 6, wobei das Sicherheitssystem Mechanismen für die Entlastung von übermäßigem Druck in der Absorberkolonne, falls der Druck in der Kolonne einen ersten Schwellenbetriebsdruck überschreitet, umfasst, wobei Bruchscheiben, die ausgelegt sind, um zu bersten, falls der Druck in der Kolonne den ersten Schwellenbetriebsdruck überschreitet, in der ganzen Absorberkolonne montiert sind und wobei der erste Schwellenbetriebsdruck auf oder unter dem Sicherheitsbetriebsdruck für die Absorberkolonne liegt.

8. Verfahren gemäß Anspruch 7, wobei der erste Schwellenbetriebsdruck zwischen 137900 Pascal (20 psig) und 413700 Pascal (60 psig) liegt.

9. Verfahren gemäß einem der Ansprüche 1 bis 8, wobei das Sicherheitssystem einen Mechanismus für die Entlastung von übermäßigem Druck stromaufwärts von den Ozongeneratoren umfasst und mindestens ein Druckentlastungsventil auf dem Trockner umfasst, wobei der Mechanismus eingestellt ist, um zu öffnen, falls der Druck des ozonangereicherten Gases vor der Einleitung in die Absorberkolonne einen zweiten Schwellenbetriebsdruck überschreitet.

10. Verfahren gemäß Anspruch 9, wobei der Druck des ozonangereicherten Gases durch das Öffnen eines Druckentlastungsventils entlastet wird und wobei die Schwelle für die Entlastung des Druckes zwischen 68950 Pascal (10 psig) und 172400 Pascal (25 psig) liegt.

11. Verfahren gemäß einem der Ansprüche 1 bis 10, wobei der Absorber 7 Stufen aufweist, wobei sich eine erste Temperaturüberwachungseinheit an jeder Stufe befindet und wobei sich eine zweite Temperaturüberwachungseinheit in 3 Stufen (112, 122, 124) befindet.

12. Verfahren gemäß einem der Ansprüche 1 bis 11, wobei die ungesättigte Carbonsäure Obtusilin-, Caprolein-, Undecen-, Laurin-, Myristolein-, Palmitolein-, Petroselin-, Olein-, Vaccen-, Octadecan-, Gadolein-, Cetolein-, Eruca-, Selacholein-, Hexacosan- oder Tricosansäure ist.

13. Verfahren gemäß einem der Ansprüche 1 bis 12, wobei die ungesättigte Carbonsäure Oleinsäure ist.

## Revendications

1. Procédé pour produire un ozonure à partir d'un acide carboxylique éthyléniquement non saturé ayant entre 8 et 30 atomes de carbone et une ou plusieurs doubles liaisons carbone à carbone non saturée comprenant les étapes de mise en contact d'un gaz enrichi en ozone avec l'acide carboxylique non saturé dans une colonne d'absorption (110) ayant une rigole (126) au niveau de sa partie supérieure (128) et un pot (130) au niveau de sa partie inférieure (132) ayant des étages distincts pour former un ozonure ; et de fonctionnement d'un système de sécurité comprenant :
la surveillance d'une condition de fonctionnement dans lequel la condition de fonctionnement inclut au moins un élément parmi une température de fonctionnement au sein de la colonne, l'écoulement de gaz enrichi en ozone ayant une concentration d'ozone de 0,5 % à 12 % à travers la colonne, la pression de fonctionnement du gaz enrichi en ozone dans la colonne, l'écoulement de réactif liquide à travers la colonne, et l'écoulement de produit hors de la colonne et l'attribution d'une valeur à la condition de fonctionnement surveillée ; et dans lequel chaque condition de fonctionnement est surveillée indépendamment ;
la comparaison de la valeur de condition de fonctionnement avec une valeur seuil pour l'au moins une condition ;
l'affection de la formation du produit en réponse au fait que la condition de fonctionnement a atteint la valeur seuil ;
dans lequel la valeur de condition de fonctionnement est une température dans la colonne d'absorption et la valeur seuil est une première température seuil entre 37 °C (100 °F) et 60 °C (140 °F) ;
et dans lequel un capteur surveille la température au sein de la colonne et un commutateur relié au capteur interrompt l'alimentation en gaz enrichi en ozone vers la colonne d'absorption en fermant une soupape de gaz enrichi en ozone et en amenant les générateurs d'ozone à s'arrêter en coupant l'alimentation en électricité vers les générateurs si la température à l'un quelconque des étages dans la colonne d'absorption, ou dans la rigole ou dans le pot est au-dessus de la première valeur de température seuil qui est entre 37 °C (100 °F) et 60 °C (140 °F) ;
et dans lequel la valeur seuil inclut en outre une deuxième température seuil qui est supérieure à la première température seuil, et la deuxième température seuil est entre 43 °C (110 °F) et 66 °C (150 °F), et dans lequel un deuxième dispositif de surveillance de la température indépendant détecte la température des étages au sein de la colonne et si la température dépasse ou atteint la deuxième température seuil mais est inférieure à la température critique, un commutateur relié au deuxième capteur de température amène les générateurs d'ozone à s'arrêter, la soupape de gaz enrichi en ozone alimentant la colonne d'absorption à se fermer, et les soupapes d'eau sont ouvertes vers l'étage où la deuxième température seuil a été dépassée et la formation du produit dans cet étage en réponse au fait que la température a atteint la deuxième valeur seuil est affectée par l'ajout direct d'eau dans la colonne d'absorption.

2. Procédé selon la revendication 1 dans lequel le système de sécurité surveille aussi l'écoulement de gaz enrichi en ozone vers la colonne d'absorption depuis le système de gaz recyclé et les composants de sécurité d'écoulement sont activés si un élément parmi la pression ou le volume du gaz enrichi en ozone, ou les deux, tombent au-dessous des valeurs seuils, dans lequel la valeur de condition de fonctionnement est une pression seuil mesurée par un capteur de pression de gaz en amont des générateurs d'ozone et/ou un volume seuil du gaz enrichi en ozone mesuré par un débitmètre gazeux au niveau de l'entrée du générateur d'ozone, dans lequel la valeur de pression de gaz seuil est une pression de gaz enrichi en ozone entre 0 kPa (0 psig) et 103 kPa (15 psig) ; et dans lequel si la pression de gaz tombe jusqu'à la valeur seuil ou au-dessous de celle-ci, les générateurs d'ozone s'arrêteront et fermeront une soupape de gaz enrichi en ozone pour interrompre l'alimentation en gaz enrichi en ozone vers la colonne d'absorption.

3. Procédé selon la revendication 1 ou 2 dans lequel le système de sécurité surveille aussi l'écoulement de réactif liquide à travers la colonne d'absorption en surveillant le volume de réactif liquide dans la rigole, et dans lequel la valeur de condition de fonctionnement est une valeur seuil de la profondeur de réactif liquide dans la rigole.

4. Procédé selon la revendication 3 dans lequel l'écoulement de réactif liquide est surveillé par un capteur pour détecter le volume du réactif liquide dans la rigole de la colonne ; et dans lequel la valeur seuil est une augmentation de la profondeur de réactif liquide dans la rigole d'au moins 5 % de la profondeur de fonctionnement normale de liquide dans la rigole de la colonne d'absorption.

5. Procédé selon l'une des revendications 3 ou 4 dans lequel si la valeur seuil est atteinte ou dépassée un signal est généré par le capteur pour arrêter les générateurs d'ozone, fermer la soupape de gaz enrichi en ozone, fermer la soupape de composé éthyléniquement non saturé, et fermer la soupape d'alimentation en diluant.

6. Procédé selon l'une des revendications 1 à 5 dans lequel le système de sécurité surveille aussi l'écoulement de produit, dans lequel le produit est de l'ozonure, des réactifs liquides non dilués et du diluant et dans lequel la valeur de condition de fonctionnement est surveillée en mesurant le niveau de liquide dans le pot de la colonne, dans lequel la valeur seuil est un niveau de produit d'un tiers de la hauteur du pot, et dans lequel si le niveau de produit atteint ou dépasse le seuil, la formation d'ozonure est affectée par l'arrêt des générateurs d'ozone par le système de sécurité, la fermeture de la soupape de gaz enrichi en ozone, la fermeture de la soupape d'acide carboxylique non saturé, et la fermeture de la soupape d'alimentation en diluant.

7. Procédé selon l'une des revendications 1 à 6 dans lequel le système de sécurité inclut des mécanismes pour décharger la pression excessive dans la colonne d'absorption si la pression dans la colonne dépasse une première pression de fonctionnement seuil, dans lequel des disques de rupture conçus pour éclater si la pression dans la colonne dépasse la première pression de fonctionnement seuil sont installés dans toute la colonne d'absorption, et dans lequel la première pression de fonctionnement seuil est au niveau ou au-dessous de la pression de fonctionnement sûre pour la colonne d'absorption.

8. Procédé selon la revendication 7 dans lequel la première pression de fonctionnement seuil est entre 137 900 pascals (20 psig) et 413 700 pascals (60 psig).

9. Procédé selon l'une des revendications 1 à 8 dans lequel le système de sécurité inclut un mécanisme pour décharger la pression excessive en amont des générateurs d'ozone et incluant au moins une soupape de décharge de pression sur le séchoir, dans lequel le mécanisme est réglé pour s'ouvrir si la pression du gaz enrichi en ozone avant l'introduction dans la colonne d'absorption dépasse une deuxième pression de fonctionnement seuil.

10. Procédé selon la revendication 9 dans lequel la pression du gaz enrichi en ozone est déchargée par l'ouverture d'une soupape de décharge de pression et dans lequel le seuil pour décharger la pression est entre 68 950 pascals (10 psig) et 172 400 pascals (25 psig).

11. Procédé selon l'une quelconque des revendications 1 à 10 dans lequel l'absorbeur comporte 7 étages, dans lequel un premier dispositif de surveillance de température est situé à chaque étage, et dans lequel un deuxième dispositif de surveillance de température est situé dans 3 étages (112, 122, 124).

12. Procédé selon l'une quelconque des revendications 1 à 11 dans lequel l'acide carboxylique non saturé est l'acide obtusilique, caproléique, undécénoïque, laurique, myristoléique, palmitoléique, pétrosélinique, oléique, vaccénique, octadécanoïque, gadoléique, cétoléique, érucique, sélacholéique, hexacosénoïque ou tricosénoïque.

13. Procédé selon l'une des revendications 1 à 12 dans lequel l'acide carboxylique non saturé est l'acide oléique.
